(19) **Europäisches Patentamt / European Patent Office / Office européen des brevets**

(11) **EP 2 434 975 B1**

(12) **EUROPEAN PATENT SPECIFICATION**

(45) Date of publication and mention of the grant of the patent:
**11.07.2018 Bulletin 2018/28**

(51) Int Cl.:
***A61B 18/12*** *(2006.01)*    ***H05H 1/24*** *(2006.01)*

(21) Application number: **09845330.1**

(22) Date of filing: **30.09.2009**

(86) International application number:
**PCT/US2009/005399**

(87) International publication number:
**WO 2010/138105 (02.12.2010 Gazette 2010/48)**

(54) **PLASMA DEVICE FOR WIDE AREA SURFACE TREATMENT OF TISSUE**

PLASMAVORRICHTUNG FÜR GROSSFLÄCHIGE OBERFLÄCHENBEHANDLUNG VON GEWEBE

DISPOSITIF À PLASMA POUR LE TRAITEMENT DE GRANDES SURFACES DE TISSU

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **29.05.2009 PCT/US2009/045708**

(43) Date of publication of application:
**04.04.2012 Bulletin 2012/14**

(73) Proprietor: **COLORADO STATE UNIVERSITY RESEARCH FOUNDATION**
**Fort Collins, CO 80522 (US)**

(72) Inventors:
  • **KOO, Il-Gyo**
    **Fort Collins**
    **Colorado 80521 (US)**
  • **SCOTT, Douglas, A.**
    **Laporte**
    **Colorado 80535 (US)**
  • **MOORE, Cameron, A.**
    **Loveland**
    **Colorado 80537 (US)**
  • **COLLINS, George, J.**
    **Fort Collins**
    **Colorado 80524 (US)**
  • **DOUGLAS, K. Antioco**
    **Fort Collins, Colorado 80521 (US)**

(74) Representative: **Soames, Candida Jane et al**
**Maschio & Soames IP Limited**
**30 Carlton Crescent**
**Southampton SO15 2EW (GB)**

(56) References cited:
EP-A1- 0 366 876        JP-A- 2003 272 896
US-A- 5 753 886         US-A1- 2003 125 727
US-A1- 2004 022 669     US-A1- 2004 140 194
US-A1- 2004 140 194     US-A1- 2007 029 500
US-A1- 2008 097 425     US-B2- 6 723 091

## Description

## BACKGROUND

*Technical Field*

The present disclosure relates to plasma devices and processes for surface processing and material removal or deposition. More particularly, the disclosure relates to an apparatus and method for generating and directing chemically reactive, plasma-generated species in a plasma device along with excited-state species (e.g., energetic photons) that are specific to the selected ingredients.

*Background of Related Art*

Electrical discharges in dense media, such as liquids and gases at or near atmospheric pressure, can, under appropriate conditions, result in plasma formation. Plasmas have the unique ability to create large amounts of chemical species, such as ions, radicals, electrons, excited-state (e.g., metastable) species, molecular fragments, photons, and the like. The plasma species may be generated in a variety of internal energy states or external kinetic energy distributions by tailoring plasma electron temperature and electron density. In addition, adjusting spatial, temporal and temperature properties of the plasma creates specific changes to the material being irradiated by the plasma species and associated photon fluxes.

Plasmas are also capable of generating photons including energetic ultraviolet photons that have sufficient energy to initiate photochemical and photocatalytic reaction paths in biological and other materials that are irradiated by the plasma photons. US2003/0125727 A1 discloses a medical instrument for controlled volumetric removal of thin tissue layer.

## SUMMARY

Plasmas have broad applicability to provide alternative solutions to industrial, scientific and medical needs, especially workpiece surface processing at low temperature. Plasmas may be delivered to a workpiece, thereby affecting multiple changes in the properties of materials upon which the plasmas impinge. Plasmas have the unique ability to create large fluxes of radiation (e.g., ultraviolet), ions, photons, electrons and other excited-state (e.g., metastable) species which are suitable for performing material property changes with high spatial, material selectivity, and temporal control. Plasmas may also remove a distinct upper layer of a workpiece but have little or no effect on a separate underlayer of the workpiece or it may be used to selectively remove a particular tissue from a mixed tissue region or selectively remove a tissue with minimal effect to adjacent organs of different tissue type.

One suitable application of the unique chemical species is to drive non-equilibrium or selective chemical reactions at or within the workpiece to provide for selective removal of only certain types of materials. Such selective processes are especially sought in biological tissue processing (e.g., mixed or multi-layered tissue), which allows for cutting and removal of tissue at low temperatures with differential selectivity to underlayers and adjacent tissues. This is particularly useful for removal of biofilms, mixtures of fatty and muscle tissue, debridement of surface layers and removing of epoxy and other non-organic materials during implantation procedures.

The plasma species are capable of modifying the chemical nature of tissue surfaces by breaking chemical bonds, substituting or replacing surface-terminating species (e.g., surface functionalization) through volatilization, gasification or dissolution of surface materials (e.g., etching). With proper techniques, material choices and conditions, one can remove one type of tissue entirely without affecting a nearby different type of tissue. Controlling plasma conditions and parameters (including S-parameters, V, I, $\Theta$, and the like) allows for the selection of a set of specific particles, which, in turn, allows for selection of chemical pathways for material removal or modification as well as selectivity of removal of desired tissue type. The present disclosure provides for a system and method for creating plasma under a broad range of conditions including tailored geometries, various plasma feedstock media, number and location of electrodes and electrical excitation parameters (e.g., voltage, current, phase, frequency, pulse condition, etc.).

The supply of electrical energy that ignites and sustains the plasma discharge is delivered through substantially conductive electrodes that are in contact with the ionizable media and other plasma feedstocks. The present disclosure also provides for methods and apparatus that utilize specific electrode structures that improve and enhance desirable aspects of plasma operation such as higher electron temperature and higher secondary emission. In particular, the present disclosure provides for porous media for controlled release of chemical reactants.

Controlling plasma conditions and parameters allows for selection of a set of specific particles, which, in turn, allows for selection of chemical pathways for material removal or modification as well as selectivity of removal of desired tissue type. The present disclosure also provides for a system and method for generating plasmas that operate at or near atmospheric pressure. The plasmas include electrons that drive reactions at material surfaces in concert with other plasma species. Electrons delivered to the material surface can initiate a variety of processes including bond scission, which enables volatilization in subsequent reactions. The electron-driven reactions act synergistically with associated fluxes to achieve removal rates of material greater than either of the reactions acting alone.

In an embodiment of the present disclosure, a plasma device includes inner and outer electrodes. The inner electrode has a substantially cylindrical tubular shape and an opening defined therethrough. The inner electrode has a proximal portion. The outer electrode has a substantially cylindrical tubular shape. The outer electrode is coaxially disposed about the proximal portion of the inner electrode. The dielectric spacer includes a substantially toroidal shape disposed between the inner and the outer electrode. The inner and outer electrodes are configured to couple to an ionizable media source configured to supply ionizable media thereto. The inner and outer electrodes are configured to couple to a power source configured to ignite the ionizable media at the plasma device to form a plasma effluent.

The plasma device may further include a porous member. The porous member is coupled to the inner electrode at a distal end thereof. The porous member is formed from a conductive porous material and is configured to disperse the plasma effluent through the conductive porous material to generate a wide-area plasma effluent. The outer electrode may be disposed only about a portion of the inner electrode.

In yet another embodiment of the present disclosure, a plasma device configured to receive ionizable media includes a dielectric housing, and first and second electrodes. The dielectric housing has a substantially cylindrical tubular shape and an opening defined therethrough. The first and second electrodes are disposed within the opening of the dielectric housing separated by a predetermined distance. The second electrode is formed from a conductive porous material and is configured to ignite the ionizable media to form a plasma having a wide-area plasma effluent.

The first electrode may be a substantially cylindrical rod formed from a conductive metal and disposed coaxially within the dielectric housing. The plasma device may further include an electrode spacer disposed between the first and second electrodes. The electrode spacer is configured to secure the first electrode to the dielectric housing. The electrode spacer includes a central opening adapted for insertion of the first electrode therethrough. The electrode spacer may include at least one flow opening for the flow of the ionizable media disposed radially around the central opening. The first electrode includes an insulative layer that covers at least a portion thereof. The insulative layer may be at least one of formed integrally with the dielectric housing and formed from a dielectric coating deposited on the first electrode. The first electrode may be formed from a conductive porous material. The plasma device may include a dielectric spacer disposed between the first and second electrodes. The dielectric spacer may be formed from a porous dielectric material.

In yet another embodiment of the present disclosure, a plasma device includes a dielectric spacer, and first and second electrodes. The dielectric spacer includes a substantially disk-like shape and is configured to couple to an ionizable media source configured to supply ionizable media thereto. The dielectric spacer is formed from a porous dielectric material configured to disperse the ionizable media. The first and second electrodes are disposed within the dielectric spacer. The first and second electrodes are configured to couple to a power source configured to ignite the ionizable media at the plasma device to form a plasma effluent. The dielectric spacer may include at least one opening to provide for a flow path of the ionizable media through the dielectric spacer. The first and second electrodes may be substantially cylindrical rods formed from a conductive metal and disposed in a parallel configuration with respect to each other and equidistant from a center of the dielectric spacer.

The present invention defines a system according to claim 1. Preferred embodiments of the present invention are defined in accordance with the dependent claims.

BRIEF DESCRIPTION OF THE DRAWINGS

The accompanying drawings, which are incorporated in and constitute a part of this specification, illustrate exemplary embodiments of the disclosure and, together with a general description of the disclosure given above, and the detailed description of the embodiments given below, serve to explain the principles of the disclosure, wherein:

Fig. 1 is a schematic diagram of a plasma system according to the present disclosure;
Fig. 2A is a perspective, cross-sectional view of a plasma device according to the present disclosure;
Figs. 2B - 2D are side, cross-sectional views of the plasma device of Fig. 2A;
Fig. 3 is a side, cross-sectional view of the plasma device of Fig. 2A;
Fig. 4 is a front, cross-sectional view of the plasma device of Fig. 2A according to the present disclosure;
Fig. 5 is an enlarged cross-sectional view of a plasma device according to the present disclosure;
Fig. 6 is an enlarged cross-sectional view of a plasma device according to one embodiment of the present disclosure;
Fig. 7 is a front, cross-sectional view of the plasma device of Fig. 2A according to the present disclosure;
Fig. 8 is a perspective, cross-sectional view of a plasma device according to the present disclosure;
Fig. 9 is a perspective, cross-sectional view of a plasma device according to the present disclosure;
Fig. 10 is a perspective, cross-sectional view of a plasma device according to the present disclosure;
Fig. 11A is a perspective, cross-sectional view of a plasma device according to the present invention;
Fig. 11B is a top view of a plasma device of Fig. 11A according to the present invention;
Fig. 11C is a top view of a plasma device of Fig. 11B according to the present invention;

Fig. 12A is a perspective, cross-sectional view of a plasma device according to the present disclosure;

Fig. 12B is a top view of a plasma device of Fig. 12A according to the present disclosure;

Fig. 13 is a perspective, cross-sectional view of a plasma device according to the present disclosure;

Fig. 14 is a schematic diagram of a plasma system according to one embodiment of the present disclosure;

Fig. 15 is a side, cross-sectional view of a plasma device according to the present disclosure;

Fig. 16 is a close-up, side view of a plasma device according to the present disclosure;

Figs. 17A and 17B are plots relating to electron emissions according to the present disclosure;

Figs. 18A, 18B, and 18C show charts illustrating several tissue effects of a plasma device according to the present disclosure;

Fig. 19 is a flow chart diagram of a method of plasma tissue treatment according to the present disclosure;

Fig. 20 is a flow chart diagram of another method of plasma tissue treatment according to the present disclosure;

## DETAILED DESCRIPTION

Plasmas are generated using electrical energy that is delivered as either direct current (DC) electricity or alternating current (AC) electricity at frequencies from about 0.1 hertz (Hz) to about 100 gigahertz (GHz), including radio frequency ("RF", from about 0.1 MHz to about 100 MHz) and microwave ("MW", from about 0.1 GHz to about 100 GHz) bands, using appropriate generators, electrodes, and antennas. Choice of excitation frequency, the workpiece, as well as the electrical circuit that is used to deliver electrical energy to the circuit affects many properties and requirements of the plasma. The performance of the plasma chemical generation, the delivery system and the design of the electrical excitation circuitry are interrelated -- as the choices of operating voltage, frequency and current levels (as well as phase) effect the electron temperature and electron density. Further, choices of electrical excitation and plasma device hardware also determine how a given plasma system responds dynamically to the introduction of new ingredients to the host plasma gas or liquid media. The corresponding dynamic adjustment of the electrical drive, such as via dynamic match networks or adjustments to voltage, current, or excitation frequency may be used to maintain controlled power transfer from the electrical circuit to the plasma.

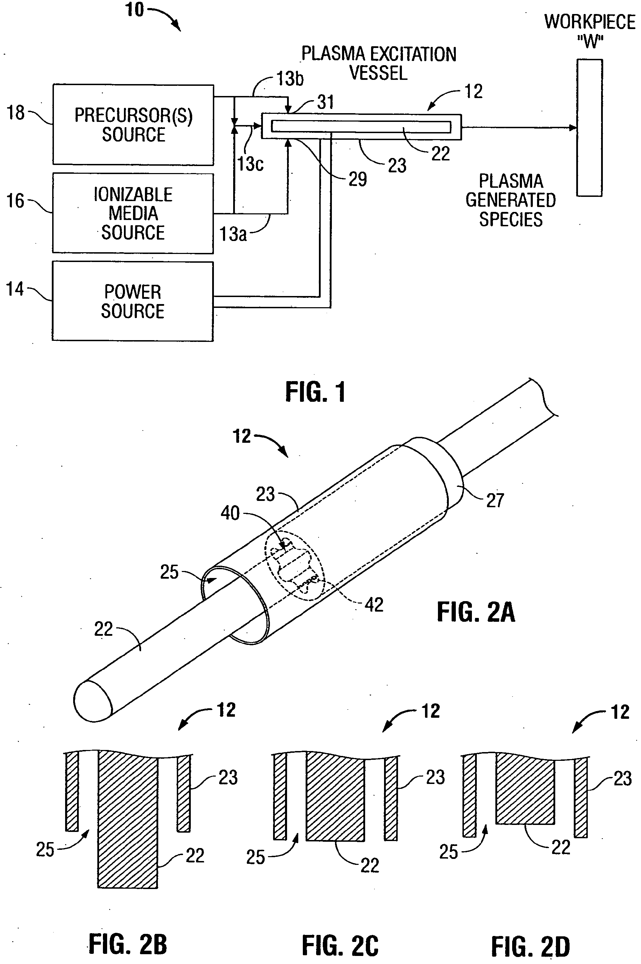

Referring initially to Fig. 1, a plasma system 10 is disclosed. The system 10 includes a plasma device 12 that is coupled to a power source 14, an ionizable media source 16 and a precursor source 18. Power source 14 includes any suitable components for delivering power or matching impedance to plasma device 12. More particularly, the power source 14 may be any radio frequency generator or other suitable power source capable of producing power to ignite the ionizable media to generate plasma. In the present invention the plasma device 12 may be utilized as an electrosurgical pencil for application of plasma to tissue. The power source 14 may be an electrosurgical generator that is adapted to supply the device 12 with electrical power at a frequency from about 0.1 MHz to about 2,450 MHz and in another embodiment from about 1 MHz to about 13.56 MHz. The plasma may also be ignited by using continuous or pulsed direct current (DC) electrical energy.

The precursor source 18 may be a bubbler or a nebulizer configured to aerosolize precursor feedstocks prior to introduction thereof into the device 12. The precursor source 18 may also be a micro droplet or injector system capable of generating predetermined refined droplet volume of the precursor feedstock from about 1 femtoliter to about 1 nanoliter in volume. The precursor source 18 may also include a microfluidic device, a piezoelectric pump, or an ultrasonic vaporizer.

The system 10 provides a flow of plasma through the device 12 to a workpiece "W" (e.g., tissue). Plasma feedstocks, which include ionizable media and precursor feedstocks, are supplied by the ionizable media source 16 and the precursor source 18, respectively, to the plasma device 12. During operation, the precursor feedstock and the ionizable media are provided to the plasma device 12 where the plasma feedstocks are ignited to form plasma effluent containing ions, radicals, photons from the specific excited species and metastables that carry internal energy to drive desired chemical reactions in the workpiece "W" or at the surface thereof. The feedstocks may be mixed upstream from the ignition point or midstream thereof (e.g., at the ignition point) of the plasma effluent, as shown in Fig. 1 and described in more detail below.

The ionizable media source 16 provides ionizable feedstock to the plasma device 12. The ionizable media source 16 is coupled to the plasma device 12 and may include a storage tank and a pump (not explicitly shown). The ionizable media may be a liquid or a gas such as argon, helium, neon, krypton, xenon, radon, carbon dioxide, nitrogen, hydrogen, oxygen, etc. and their mixtures, and the like, or a liquid. These and other gases may be initially in a liquid form that is gasified during application.

The precursor source 18 provides precursor feedstock to the plasma device 12. The precursor feedstock may be either in solid, gaseous or liquid form and may be mixed with the ionizable media in any state, such as solid, liquid (e.g., particulates or droplets), gas, and the combination thereof. The precursor source 18 may include a heater, such that if the precursor feedstock is liquid, it may be heated into gaseous state prior to mixing with the ionizable media.

In one embodiment, the precursors may be any chemical species capable of forming reactive species such as ions, electrons, excited-state (e.g., metastable) species, molecular fragments (e.g., radicals) and the like, when ignited by electrical energy from the power source 14 or when undergoing collisions with particles (electrons, photons, or other energy-bearing species of limited and selective chemical reactivity) formed from ionizable media 16. More specifically, the precursors may include various reactive functional groups, such as acyl halide, alcohol, aldehyde, alkane, alkene, amide, amine, butyl, carboxlic, cyanate, isocyanate, ester, ether, ethyl, halide, haloalkane, hydroxyl, ketone, methyl, nitrate, nitro, nitrile, nitrite, nitroso, peroxide, hydroperoxide, oxygen, hydrogen, nitrogen, and combination thereof. In embodiments, the chemical precursors may be water, halogenoalkanes, such as dichloromethane, tricholoromethane, carbon tetrachloride, difluoromethane, trifluoromethane, carbon tetrafluoride, and the like; peroxides, such as hydrogen peroxide, acetone peroxide, benzoyl peroxide, and the like; alcohols, such as methanol, ethanol, isopropanol, ethylene glycol, propylene glycol, alkalines such as NaOH, KOH, amines, alkyls, alkenes, and the like. Such chemical precursors may be applied in substantially pure, mixed, or soluble form.

The precursors and their functional groups may be delivered to a surface to react with the surface species (e.g., molecules) of the workpiece "W." In other words, the functional groups may be used to modify or replace existing surface terminations of the workpiece "W." The functional groups react readily with the surface species due to their high reactivity and the reactivity imparted thereto by the plasma. In addition, the functional groups are also reacted within the plasma volume prior to delivering the plasma volume to the workpiece.

Some functional groups generated in the plasma can be reacted *in situ* to synthesize materials that subsequently form a deposition upon the surface. This deposition may be used for stimulating healing, killing bacteria, and increasing hydrophilic or hydroscopic properties. In addition, deposition of certain function groups may also allow for encapsulation of the surface to achieve predetermined gas/liquid diffusion, e.g., allowing gas permeation but preventing liquid exchange, to bond or stimulate bonding of surfaces, or as a physically protective layer.

The precursor source 18 and the ionizable media source 16 may be coupled to the plasma device 12 via tubing 13a and 13b, respectively. The tubing 13a and 13b may be combined into tubing 13c to deliver a mixture of the ionizable media and the precursor feedstock to the device 12 at a proximal end thereof. This allows for the plasma feedstocks, e.g., the precursor feedstock and the ionizable gas, to be delivered to the plasma device 12 simultaneously prior to ignition of the mixture therein.

In another embodiment, the ionizable media source 16 and the precursors source 18 may be coupled to the plasma device 12 via the tubing 13a and 13b at separate connections, e.g., the first connection 31 and a second connection 29, respectively, such that the mixing of the feedstocks occurs within the plasma device 12 upstream from the ignition point. In other words, the plasma feedstocks are mixed proximally of the ignition point, which may be any point between the respective sources 16 and 18 and the plasma device 12, prior to ignition of the plasma feedstocks to create the desired mix of the plasma effluent species for each specific surface treatment on the workpiece "W."

In a further embodiment, the plasma feedstocks may be mixed midstream, e.g., at the ignition point or downstream of the plasma effluent, directly into the plasma. More specifically, the first and second connections 31, 29 may be coupled to the device 12 at the ignition point, such that the precursor feedstocks and the ionizable media are ignited concurrently as they are mixed (Fig. 1). It is also envisioned that the ionizable media may be supplied to the device 12 proximally of the ignition point, while the precursor feedstocks are mixed therewith at the ignition point.

In a further illustrative embodiment, the ionizable media may be ignited in an unmixed state and the precursors may be mixed directly into the ignited plasma. Prior to mixing, the plasma feedstocks may be ignited individually. The plasma feedstock is supplied at a predetermined pressure to create a flow of the medium through the device 12, which aids in the reaction of the plasma feedstocks and produces a plasma effluent. The plasma according to the present disclosure is generated at or near atmospheric pressure under normal atmospheric conditions.

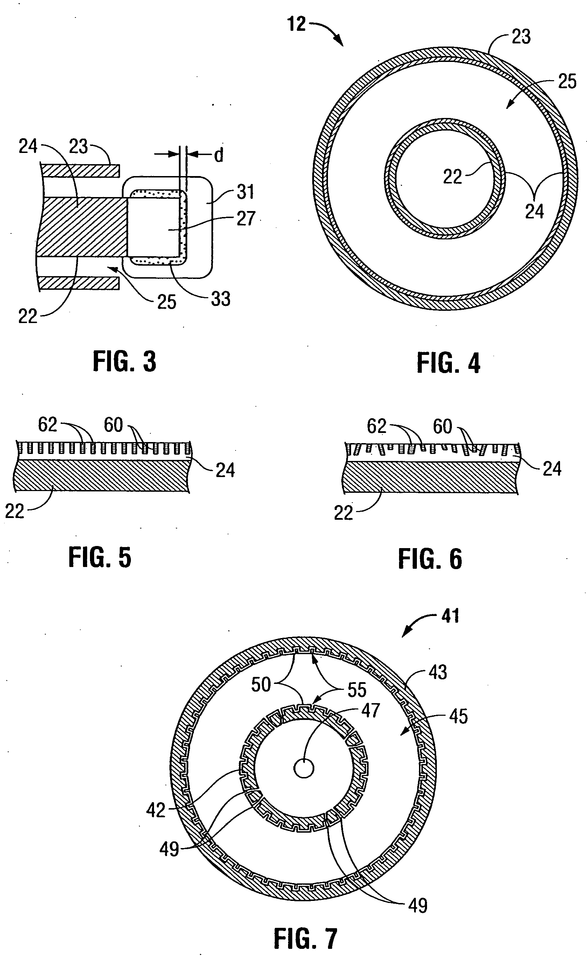

With reference to Figs. 1-3, the device 12 includes an inner electrode 22 disposed coaxially within an outer electrode 23. As shown in Fig. 2A, the outer electrode 23 has a substantially cylindrical tubular shape having an opening 25 (Fig. 3) defined therein. The inner electrode 22 has a substantially cylindrical shape (e.g., rod-shaped). The electrodes 22 and 23 may be formed from a conductive material suitable for ignition of plasma such as metals and metal-ceramic composites. In one embodiment, the electrodes 22 and 23 may be formed from a conductive metal including a native oxide or nitride compound disposed thereon.

The device 12 also includes an electrode spacer 27 disposed between the inner and outer electrodes 22 and 23. The electrode spacer 27 may be disposed at any point between the inner and outer electrodes 22 and 23 to provide for a coaxial configuration between the inner and outer electrodes 22 and 23. The electrode spacer 27 includes a central opening 40 adapted for insertion of the inner electrode 22 therethrough and one or more flow openings 42 disposed radially around the central opening 40 to allow for the flow of ionizable media and precursors through the device 12. The electrode spacer 27 may be frictionally fitted to the electrodes 22 and 23 to secure the inner electrode 22 within the outer electrode 23. In another embodiment, the electrode spacer 27 is slidably disposed over the inner electrode 22. In one illustrative embodiment, the electrode spacer 27 may be formed from a dielectric material, such as ceramic, to provide capacitive coupling between the inner and outer electrodes 22 and 23.

As shown in Fig. 2B, distal end of the inner electrode 22 may extend past the distal end of the outer electrode 23. In another embodiment, as shown in Figs. 2C and 2D, the inner electrode 22 may be fully enclosed by the outer electrode 23. In particular, the distal end the inner electrode 22 may be flush with the distal end of the outer electrode 23 (Fig. 2C). In a further embodiment, the inner electrode 22 may be recessed within the outer electrode 23 (e.g., distal end of the inner electrode 22 is within the opening 25 as shown in Fig. 2D).

The extended distance of the inner electrode 22 relative to the outer electrode 23 may be adjusted to achieve a desired spatial relationship between the electrodes 22 and 23. In one embodiment, the electrode spacer 27 is secured to the outer electrode 23 but is slidably disposed over the inner electrode 22. In other words, the inner electrode 22 may move through the opening 40. This allows for the outer electrode 23 and the electrode spacer 27 to be longitudinally movable along the inner electrode 22 thereby controlling the exposure of the distal end of the inner electrode 22. In another embodiment, the inner and outer electrodes 22 and 23 may be fixated in a coaxial configuration using other fixation mechanisms (e.g., clamps) that allow for adjustment of the exposure distance of the inner electrode 22.

One of the electrodes 22 and 23 may be an active electrode and the other may be a neutral or return electrode to facilitate in RF energy coupling. Each of the electrodes 22 and 23 are coupled to the power source 14 that drives plasma generation and electron sheath formation close to the inner electrode 22, such that the energy from the power source 14 may be used to ignite the plasma feedstocks flowing through the device 12. More specifically, the ionizable media and the precursors flow through the device 12 through the opening 25 (e.g., through the electrode spacer 27 and between the inner and outer electrodes 22 and 23). The inner electrode 22 may also include one or more openings (not explicitly shown) therethrough to facilitate the flow of ionizable media and the precursors. When the electrodes 22 and 23 are energized, the plasma feedstocks are ignited and form a plasma effluent which is emitted from the distal end of the device 12 onto the workpiece "W."

As shown in Fig. 3, the inner electrode 22 includes a coating 24 that covers at least a portion of the inner electrode 22 leaving an exposed (e.g., uninsulated or uncoated) distal portion 27 of the inner electrode 22 uninsulated. In another embodiment, the coating 24 may be disposed on the outer electrode 23 as discussed in more detail below with respect to Figs. 4-7 and 16.

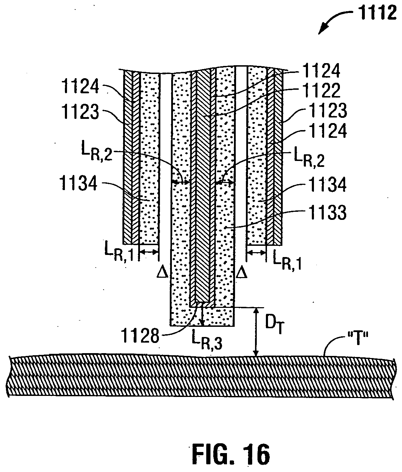

The coating 24 may be formed from an insulative or semiconductive material deposited as a film unto the inner conductor (e.g., atomic layer deposition) or as a dielectric sleeve or layer. In one illustrative embodiment, the insulative cover 24 may be a native metal oxide. The coating 24 limits the plasma action to the distal portion 27 and provides for the creation of a plasma effluent 31 having an energetic electron sheath layer 33. The sheath layer 33 has a reaching distance "d" from about 1 to about 10 mm, suitable for contacting the sheath layer 33 to the workpiece "W" to promote volatilization and/or modification of chemical bonds at the surface thereof as discussed in more detail below with respect to Figs. 16 - 24.

In addition, the coating 24 provides for capacitive coupling between the inner and outer electrodes 22 and 23. The resulting capacitive circuit element structure provides for a net negative bias potential at the surface of the inner electrode 22, which attracts the ions and other species from the plasma effluent. These species then bombard the coating 24 and release the electrons generating the sheath layer 33.

The sheath layer 33 is generated in part by the materials of the electrodes 22 and 23 and in particular by the coating 24. Materials having high secondary electron emission property, $\gamma$, in response to ion and/or photon bombardment are suitable for this task. Such materials include insulators and/or semiconductors. These materials have a relatively high $\gamma$, where $\gamma$ represents the number of electrons emitted per incident bombardment particle. Thus, metals generally have a low $\gamma$ (e.g., less than 0.1) while insulative and semiconductor materials, such as metallic oxides have a high $\gamma$, from about 1 to about 10 with some insulators exceeding a value of 20. Thus, the coating 24 acts as a source of secondary emitted electrons, in addition to limiting the plasma to the distal end of the inner electrode 22.

Secondary electron emission, $\gamma$, may be described by the formula (1):

$$(1) \qquad \gamma = \Gamma_{secondary} / \Gamma_{ion}$$

In formula (1) $\gamma$ is the secondary electron emission yield or coefficient, $\Gamma_{secondary}$ is the electron flux, and $\Gamma_{ion}$ is the ion flux. Secondary emission occurs due to the impacts of plasma species (ions) onto the coating 24 when the ion impact collisions have sufficient energy to induce secondary electron emission, thus generating $\gamma$-mode discharges. Generally discharges are said to be in $\gamma$-mode when electron generation occurs preferentially at electrode surfaces (i.e., $\gamma > 1$) instead of in the gas (an $\alpha$-mode discharge). In other words, per each ion colliding with the coating 24, a predetermined number of secondary electrons are emitted. Thus, $\gamma$ may also be thought of as a ratio of the $\Gamma_{secondary}$ (e.g., the electron flux) and $\Gamma_{ion}$ (e.g., the ion flux).

These ion collisions with the surface of the coating 24, in turn, provide sufficient energy for secondary electron emission to generate $\gamma$ discharges. The ability of coating materials such as coating 24 to generate $\gamma$ discharges varies with several parameters, with the most influence due to the choice of materials having a high $\gamma$ as discussed above. This property allows coatings 24 to act as a source of secondary emitted electrons or as a catalytic material to enhance selected chemical reaction paths.

Over time the coating 24 may thin or be removed during the plasma operation. In order to maintain the coating 24 to continually provide a source of secondary emitted electrons, the coating 24 may be continually replenished during the plasma operation. This may be accomplished by adding species that reformulate the native coating 24 on the inner and outer electrodes 22 and 23. In one embodiment, the precursor source 18 may provide either oxygen or nitrogen gas to the device 12 to replenish to oxide or nitride coating.

Generation of the sheath layer 33 is also controlled by the supply of the ionizable media and the precursors. Ionizable media and the precursors are selected that are relatively transparent to the energetic electrons released during secondary emission from the surface of the inner electrode 22. As stated above, the plasma is generated at atmospheric pressure. Due to the increased entropy at such pressure, the generated electrons undergo a multitude of collisions in a relatively short period of time and space forming the sheath layer 33.

The thickness of the sheath layer 33 is defined by a formula (2):

$$(2)\ \text{Thickness} = 1/N\sigma$$

In formula (2), N is the number of scattering centers, which may be the molecules of the ionizable media, the precursors and the atmospheric gases. Thus, N defines the media density. The variable, $\sigma$, is the average particle cross-section of the scattering centers. The thickness of the sheath layer 33 is inversely proportional to the product of N and $\sigma$. Thus, decreasing N and $\sigma$ allows for achieving a thicker sheath layer 33. A lower $\sigma$ may be provided by using specific ionizable media compounds with molecules having a low cross-section, such as hydrogen and helium. The variable N may be lowered by heating the ionizable media to reduce the gas density and limiting the amount of media provided to the lowest amount needed to sustain the plasma reaction.

The present disclosure also relates to systems and methods for generating plasma effluents having the energetic electron sheath layer having a reaching distance "d." The sheath layer 33 is produced by the combination of disclosed electrode structures, specific gas species, electrode materials, proper excitation conditions, and other media parameters. The propagation of energetic electron for mm-sized distances provides for practical applications on a variety of surfaces, such as modification of chemical bonds on the surface and volatilization of surface compounds.

In another embodiment as shown in Figs. 4-6, the coating 24 is disposed on the outer surface of the inner electrode 22 and on the inner surface of the outer electrode 23. In other words, the surfaces of the inner and outer electrodes 22 and 23 facing the opening 25 include the coating 24. In one embodiment, the coating 24 may cover the entire surface of the inner and outer electrodes 22 and 23 (e.g., outer and inner surface thereof, respectively). In another embodiment, the coating 24 may cover only a portion of the electrodes 22 and 23, such as a distal, proximal (e.g., Fig. 3 illustrates an uncoated distal portion 27) or middle portions thereof.

The coating 24 may be a native oxide, or a native nitride of the metal from which the inner and outer electrodes are formed, or may be a deposited layer or a layer formed by ion implantation. In one illustrative embodiment, the inner and outer electrodes 22 and 23 are formed from an aluminum alloy and the coating 24 is aluminum oxide ($Al_2O_3$) or aluminum nitride (AlN). In another illustrative embodiment, the inner and outer electrodes 22 and 23 are formed from a titanium alloy and the coating 24 is titanium oxide ($TiO_2$) or titanium nitride (TiN).

The inner and outer electrodes 22 and 23 and the coating 24 may also be configured as a heterogeneous system. The inner and outer electrodes 22 and 23 may be formed from any suitable electrode substrate material (e.g., conductive metal or a semiconductor) and the coating 24 may be disposed thereon by various coating processes. The coating 24 may be formed on the inner and outer electrodes 22 and 23 by exposure to an oxidizing environment, anodization, electrochemical processing, ion implantation, or deposition (e.g., sputtering, chemical vapor deposition, atomic layer deposition, etc.).

In another embodiment the coating 24 on electrodes 22 and 23 may be different on each electrode and may serve separate purposes. One coating 24 (e.g., on the electrode 22) can be selected to promote increased secondary electron emission while coating 24 on the other electrode (e.g., electrode 23) can be selected to promote specific chemical reactions (e.g., act as a catalyst).

As shown in Figs. 5 and 6, the coating 24 may also include a plurality of nanostructure pores 60, which may be arranged in a predetermined (e.g., unidirectional) form (Fig. 5) or in a random configuration (Fig. 6). Pores 60 may be formed during the coating processes discussed above. In one illustrative embodiment, the pores 60 may be treated to include one or more types of precursor feedstock 62 disposed therein. This allows for feeding of the precursor feedstock 62 directly into the plasma effluent either as a substitute for the precursor source 18 or in conjunction therewith. The precursor feedstock 62 may be the precursors discussed above with respect to the precursor source 18. In one embodiment, the precursor feedstock 62 may be a catalyst suitable for initiation of the chemical reactions between the precursor feedstock supplied from the precursor source 18 and the plasma.

Fig. 7 shows a side cross-sectional view of a plasma device 41 having an inner electrode 42 disposed coaxially within an outer electrode 43. The outer electrode 43 has a substantially cylindrical tubular shape having an opening 45 defined therein. The inner electrode 42 has a substantially cylindrical shape and may be fully enclosed by the outer electrode 43 or extend past the distal end of the outer electrode 43.

The device 41 also includes an electrode spacer (not explicitly shown) disposed between the inner and outer electrodes 42 and 43, similar to the electrode spacer 27. The electrode spacer may be disposed at any point between the inner and outer electrodes 42 and 43 to provide for a coaxial configuration between the inner and outer electrodes 42 and 43. The electrode spacer may be frictionally fitted to the electrodes 42 and 43 to secure the inner electrode 42 within the outer electrode 43. In one illustrative embodiment, the electrode spacer may be formed from a dielectric material, such as ceramic, to provide for capacitive coupling between the inner and outer electrodes 42 and 43.

Each of the inner and outer electrodes 42 and 43 may include a plurality of geometrical arrangements. In one embodiment, as shown in Fig. 7, the inner and outer electrodes 42 and 43 include a plurality of grooves 55 disposed on the surface thereof. The grooves 55 enhance the local electrical fields along the inner and outer electrodes 42 and 43. The grooves 55 may also be covered by a groove coating 50, which is substantially similar to the coating 24 for similar functional purposes. The grooves 55 are disposed on the outer surface of the inner electrode 42 and on the inner surface of the outer electrode 43. The inner and outer electrodes 42 and 43 and the coating 50 may be formed from the materials discussed above with respect to the inner and outer electrodes 22 and 23. In one embodiment, the groove coating 50 may be formed from substantially similar materials as the coating 24, namely, a combination of aluminum, magnesium, or titanium metals, and oxides or nitrides thereof.

The grooves 55 may be arranged in parallel with a longitudinal axis 47 defined by the inner and outer electrodes 42 and 43. In another embodiment, the grooves 45 may be arranged in a spiral configuration (e.g., rifled) on the inner and outer electrodes 42 and 43.

The inner electrode 43 may also include one or more side vents 49 to allow for additional gas flow into the opening 45.

The present disclosure provides for a variety of plasma device embodiments and configurations suitable for wide area plasma treatment of tissue. Common to the disclosed embodiments is the uniform dispersion of plasma feedstocks in the vicinity of both active and return electrodes employed. In one embodiment, the plasma conditions provide for a plasma media that flows in a laminar form within plasma device 12.

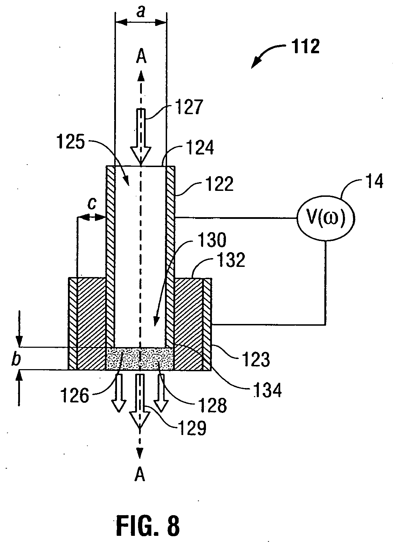

Fig. 8 shows a plasma device 112 includes an inner electrode 122 having a substantially cylindrical tubular shape having an opening 125 defined therethrough. The inner electrode 122 has a distal end 126 and proximal end 124 that is coupled to the ionizable media source 16 and the precursor source 18 (Fig. 1). The inner electrode 122 is also coupled to a porous member 128 at the distal end 126. The porous member 128 disperses the plasma passing through the inner electrode 122 to generate a wide-area plasma effluent 129. The inner electrode 122 may have an inner diameter $a$ of 10 cm or less. The porous member 128 may be formed from sintered or metal glass, ceramic mesh, and other porous materials suitable for dispersion of gas. The porous member 128 may have a thickness b from about 0.1 to about 1.0 cm.

The plasma device 112 also includes an outer electrode 123 that also has a substantially cylindrical tubular or annular shape having a larger diameter than the diameter of the inner electrode 122. The inner and outer electrodes 122 and 123 are concentrically disposed about a longitudinal axis A-A. The outer electrode 123 has a shorter length than the inner electrode 122 and is disposed coaxially about the inner electrode 122. In particular, the outer electrode 123 encloses a distal portion 130 of the inner electrode 122 and the porous member 128.

The electrodes 122 and 123 may be formed from an electrically conductive or semi-conducting material suitable for ignition of plasma such as metals and metal-ceramic composites. In one embodiment, the electrodes 122 and 123 may be formed from a conductive metal including a native oxide or nitride compound disposed thereon.

The plasma device 112 also includes a dielectric spacer 132 having puck-like or toroidal shape. The dielectric spacer 132 includes an opening 134 through the center thereof that is adapted for insertion of the inner electrode 122 therethrough. The dielectric spacer 132 is disposed between the inner and outer electrodes 122 and 123. In one embodiment, the dielectric spacer 132 may be frictionally fitted to the electrodes 122 and 123 to secure the inner electrode 122 within the outer electrode 123. The dielectric spacer may have a thickness c from about 0.1 to about 1.0 cm (e.g., gauge). In one illustrative embodiment, the electrode spacer 132 may be formed from a dielectric material, such as a thin ceramic, to provide capacitive coupling between the inner and outer electrodes 122 and 123.

One of the electrodes 122 and 123 may be an active electrode and the other may be a neutral or return electrode to facilitate in RF energy coupling. Each of the electrodes 122 and 123 are coupled to the power source 14 that drives plasma generation, such that the energy from the power source 14 may be used to ignite and sustain the plasma in feedstocks 127 flowing through the device 112 (e.g., through the opening 125).

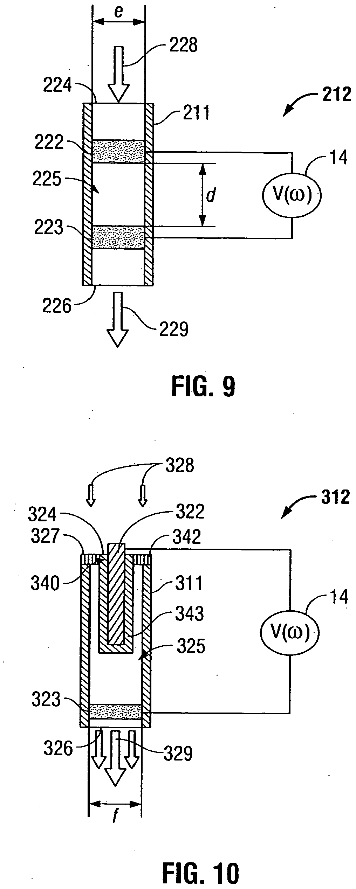

Fig. 9 shows another illustrative embodiment of a plasma device 212 which includes a housing 211 enclosing a first electrode 222 and a second electrode 223 separated by a predetermined distance d, which may be from about 0.1 cm to about 1 cm. The first electrode 222 is proximal of the second electrode 223 with respect to the supplied plasma feedstocks. The housing 211 has a substantially cylindrical tubular shape having an opening 225 defined therethrough. The housing 211 is formed from a dielectric material that insulates the first and second electrodes 222 and 223. The housing 211 may have an inner diameter e of 10 cm or less.

The plasma device 212 includes a distal end 226 and proximal end 224 that is coupled to the ionizable media source 16 and the precursor source 18. The first and second electrodes 222 and 223 are formed from conductive porous material, such as metal, metal-ceramic and semi-conducting composite meshes, porous sintered solids, and the like to permit the flow of plasma feedstocks 228 therethrough. The first and second electrodes 222 and 223 disperse the plasma passing through the housing 211 to generate a dispersed wide-area plasma effluent 229.

One of the electrodes 222 and 223 may be an active electrode and the other may be a neutral or return electrode to facilitate in RF energy coupling. Each of the electrodes 222 and 223 are coupled to the power source 14 that drives plasma generation, such that the energy from the power source 14 may be used to ignite the plasma feedstocks flowing through the device 212. The electrodes 222 and 223 are separated by a predetermined distance and are capacitively or inductively coupled through the plasma effluent 229 and the housing 211. More specifically, the ionizable media and the precursors flow through the device 212 through the chambered opening 225. As energy is applied to the electrodes 222 and 223, the plasma feedstocks are ignited to form the plasma effluent 229.

Fig. 10 shows another illustrative embodiment of a plasma device 312 which includes a housing 311 enclosing a first electrode 322 and a second electrode 323. The housing 311 has a substantially cylindrical tubular shape having a chambered opening 325 defined therethrough. The housing 311 is formed from a dielectric material that insulates the first and second electrodes 322 and 323. The housing 311 may have an inner diameter of 10 cm or less.

The plasma device 312 includes a distal end 326 and proximal end 324 that is coupled to the ionizable media source 16 and the precursor source 18. The first electrode 322 may be a cylindrical rod formed from a conductive metal (e.g., aluminum alloy) or semiconductive material, disposed coaxially within the housing 311.

The plasma device 312 also includes an electrode spacer 327 disposed between first electrode 322 and the housing 311. The electrode spacer 327 is substantially similar to the electrode spacer 27 and may include a central opening 340 adapted for insertion of the inner electrode 322 therethrough and one or more flow openings 342 disposed radially around the central opening to allow for the flow of plasma feedstocks 328 (e.g., ionizable media and precursors) through the device 312. The electrode spacer 327 may be frictionally fitted to the housing 311 and the first electrode 322 to secure the first electrode 22 within the housing 311. In one illustrative embodiment, the electrode spacer 327 may be formed from a dielectric material, such as ceramic. In another embodiment, the electrode spacer 327 may be formed integrally with the housing 311.

The first electrode 322 also includes an insulative layer 343, which may be formed integrally with the housing 311 and the electrode spacer 327. In another illustrative embodiment, the layer 343 may be formed from a dielectric material deposited as a film unto or grown on the inner conductor via processes including, but not limited to, sputtering, chemical vapor (e.g., atomic layer deposition, evaporation, electrochemical methods, or ion implantation.). The insulative layer 343 may also be a native metal oxide or nitride if the first electrode 332 is formed from a suitable alloy, such as aluminum and titanium. In particular, the first electrode 322 may be formed from an aluminum alloy and the layer 342 may be aluminum oxide ($Al_2O_3$) or aluminum nitride (AlN). In another illustrative embodiment, the first electrode 322 may be formed from a titanium alloy and the layer 342 may be titanium oxide ($TiO_2$) or titanium nitride (TiN).

The second electrode 323 is formed from a conductive or semiconductive porous material, such as metal and metal-ceramic composite meshes, porous sintered solids and the like to permit the flow of plasma feedstocks 328 therethrough. The second electrode 323 also disperses the plasma passing through the housing 311 to generate a wide-area plasma effluent 329.

One of the electrodes 322 and 323 may be an active electrode and the other may be a neutral or return electrode to facilitate in RF energy coupling. Each of the electrodes 322 and 323 are coupled to the power source 14 that drives plasma generation, such that the energy from the power source 14 may be used to ignite the plasma feedstocks flowing through the device 312. The electrodes 322 and 323 are capacitively or inductively coupled through the plasma effluent 329 and the housing 311. More specifically, the ionizable media and the precursors flow through the device 312 through the chambered opening 325. As energy is applied to the electrodes 322 and 323, the plasma feedstocks are ignited to form the plasma effluent 329.

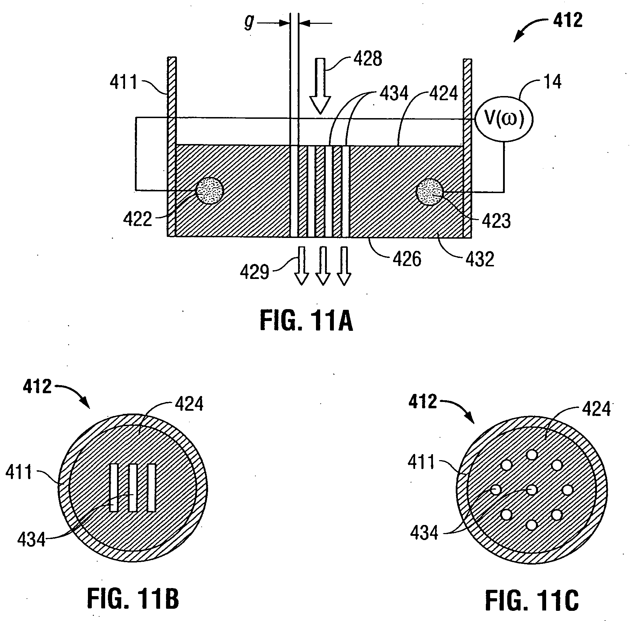

Figs. 11A - C show another illustrative embodiment of a plasma device 412 which includes a housing 411 and a dielectric spacer 432 having disk-like or toroidal shape disposed within the housing 411. The dielectric spacer 432 may be frictionally fitted to the housing 411. In one illustrative embodiment, the dielectric spacer 432 may be formed integrally with the housing 411.

The dielectric spacer 432 includes a bottom surface 426 and a top surface 424 that is coupled to the ionizable media source 16 and the precursor source 18 (Fig. 1). The electrode spacer 432 may be formed from a dielectric material, such as ceramic, plastic, and the like. The dielectric spacer 432 includes one or more openings 434 through the center thereof to allow for the flow of plasma feedstocks 428 therethrough. In the present invention, the dielectric spacer 432 is formed from a porous dielectric media suitable for allowing gases to flow therethrough thereby obviating the need for openings 434. The multiple openings 434 and/or porous nature of the dielectric spacer 432 provide for dispersion of the plasma passing therethrough to generate a wide-area plasma effluent 429. The openings 434 may be of various shapes and sizes. Fig. 11B shows the openings 434 as slits formed in the dielectric spacer 432. Fig. 11C shows the openings 434 as substantially cylindrical lumens. At its widest thickness *g*, the openings 434 may be from about 0.1 cm to about 1.0 cm. In the present invention the dielectric spacer 432 has a substantially disk-like shape and is formed from a porous dielectric material configured to disperse the ionizable media.

The plasma device 412 also includes first and second electrodes 422 and 423 disposed within the dielectric spacer 432. The first and second electrodes 422 and 423 may be cylindrical rods, formed from a conductive metal (e.g., aluminum alloy) and may be inserted into the dielectric spacer 432 in parallel configuration and equidistant from the center of the dielectric spacer 432. The dielectric spacer 432 provides capacitive coupling between the inner and outer electrodes 422 and 423. In one embodiment electrodes 422 and 423 may have one or more regions that form and present sharpened protuberances toward openings 434 to increase the local electric fields.

In the present invention one of the electrodes 422 and 423 is an active electrode and the other is a neutral or return electrode to facilitate in RF energy coupling. Each of the electrodes 422 and 423 are coupled to the power source 14 that drives plasma generation, such that the energy from the power source 14 may be used to ignite the plasma feedstocks flowing through the device 412. The electrodes 422 and 423 are capacitively coupled through the plasma effluent 429 and the dielectric spacer 432. More specifically, the ionizable media and the precursors flow through the device 412 through the openings 434. As energy is applied to the electrodes 422 and 423, the plasma feedstocks are ignited to form the plasma effluent 429.

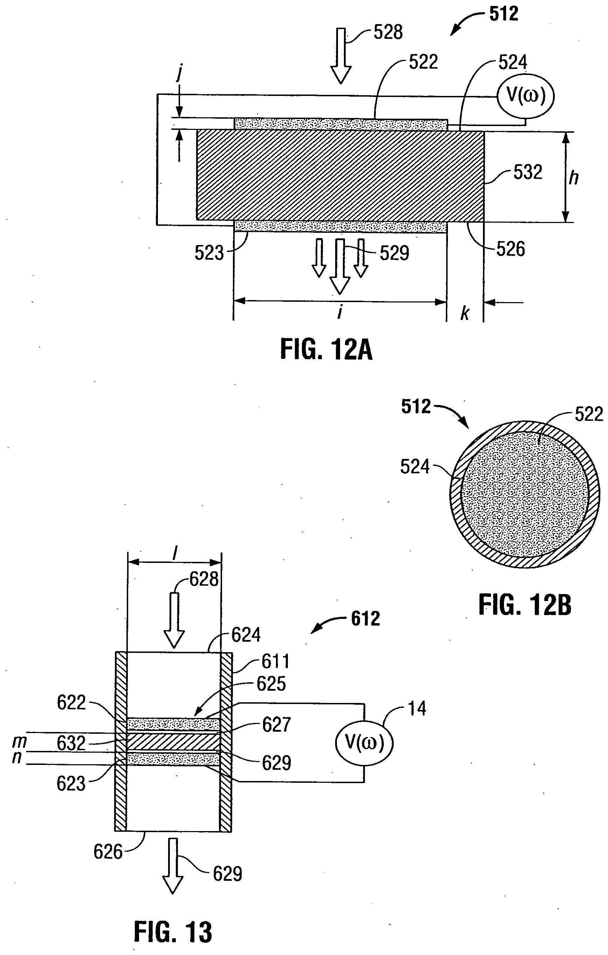

Figs. 12A - B show another illustrative embodiment of a plasma device 512 which includes a dielectric spacer 532 having a substantially disk shape. The plasma device 512 includes a bottom surface 526 and a top surface 524 that is coupled to the ionizable media source and the precursor source 18 (Fig. 1). The electrode spacer 532 may be formed from a dielectric material, such as ceramic, plastic, and the like. In one illustrative embodiment, the dielectric spacer 532 may be formed from a porous dielectric media suitable for allowing gases to flow therethrough, or otherwise have open ports to allow flow of plasma feedstocks 528 through the plasma device 512. The electrode spacer 532 may have a thickness h from about 0.1 cm to about 1.0 cm.

The plasma device 512 also includes first and second electrodes 522 and 523. The first and second electrodes 522 and 523 may also have a disk or plate shape and are disposed on the top and bottom surfaces 524 and 526, respectively. The first and second electrodes 522 and 523 are formed from a conductive or semiconductive porous material, such as metal and metal-ceramic composite meshes, porous sintered solids, and the like to permit the flow of plasma feedstocks 528 therethrough, or otherwise have open ports to allow flow of plasma feedstocks 528 through the plasma device 512. The first and second electrodes 522 and 523 may have a diameter *i* from about 0.1 cm to about 1.0 cm and a thickness *j* from about 0.1 cm to about 1.0 cm.

The dielectric spacer 532 may have a larger diameter extending outside the periphery of the first and second electrodes 522 and 523, such that a border *k* is formed, which may be from about 0.1 cm to about 1.0 cm. This configuration enhances capacitive coupling between the inner and outer electrodes 522 and 523. One or both of electrodes 522 and 523 may also be formed into predetermined surface shapes and features to induce effects such as inductive coupling. The porous nature of the dielectric spacer 532 in conjunction with the first and second electrodes 522 and 523 provides for dispersion of the plasma passing therethrough to generate a wide-area plasma effluent 529.

One of the electrodes 522 and 523 may be an active electrode and the other may be a neutral or return electrode to facilitate in RF energy coupling. Each of the electrodes 522 and 523 are coupled to the power source 14 that drives plasma generation, such that the energy from the power source 14 may be used to ignite the plasma feedstocks 528 flowing through the device 512. The ionizable media and the precursors flow through the device 512 and as energy is applied to the electrodes 522 and 523, the plasma feedstocks are ignited to form the plasma effluent 529.

Fig. 13 shows another illustrative embodiment of a plasma device 612, which is a combination of the plasma device 212 of Fig. 9 and plasma device 512 of Fig. 12. The plasma device 612 includes a housing 611 enclosing a dielectric spacer 632 having disk shape, a first electrode 622 and a second electrode 623. The housing 611 may have an inner diameter *l* of 10 cm or less. The plasma device 612 includes a bottom surface 626 and a top surface 624 that is coupled to the ionizable media source 16 and the precursor source 18 (Fig. 1). The dielectric spacer 632 may be formed from a dielectric material, such as ceramic, plastic, and the like. In one illustrative embodiment, the dielectric spacer 632 may be formed from a porous dielectric media suitable for allowing gases to flow therethrough. The dielectric spacer 632 has a thickness *m* from about 0.1 cm to about 1.0 cm.

The first and second electrodes 622 and 623 may also have a disk or plate shape and are disposed on the top and bottom surfaces 627 and 629, respectively. The first and second electrodes 622 and 623 have a thickness n from about 0.1 cm to about 1.0 cm and are formed from a conductive porous material, such as metal and metal-ceramic composite meshes, porous sintered solids, and the like to permit the flow of plasma feedstocks 628 therethrough. The porous nature of the dielectric spacer 632 in conjunction with the first and second electrodes 622 and 623 provides for dispersion of the plasma passing therethrough to generate a wide-area plasma effluent 629. The dielectric spacer 632 also provides for capacitive coupling between the inner and outer electrodes 622 and 623.

One of the electrodes 622 and 623 may be an active electrode and the other may be a neutral or return electrode to facilitate in RF energy coupling. Each of the electrodes 622 and 623 are coupled to the power source 14 that drives plasma generation, such that the energy from the power source 14 may be used to ignite and sustain the plasma feedstocks 628 flowing through the device 612. In one embodiment one electrode may be a solid and the second electrode formed into a spiral or other highly inductive form to achieve inductive coupling. The ionizable media and the precursors 628 flow through the device 612 and as energy is applied to the electrodes 622 and 623, the plasma feedstocks are ignited to form the plasma effluent 629.

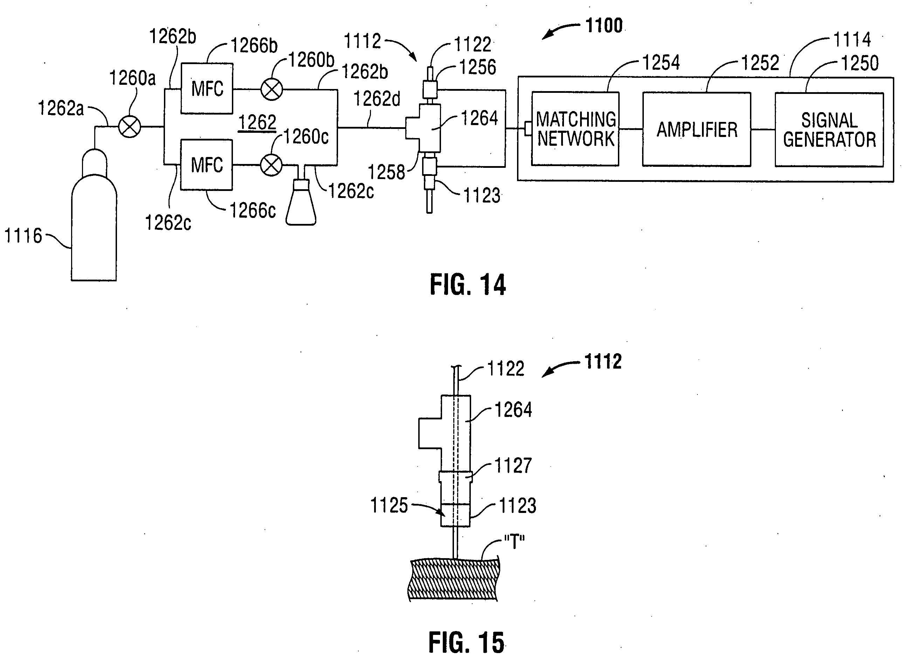

Figs. 14 and 15 show an illustrative embodiment of a plasma system 1100. The system 1100 includes a plasma device 1112 that is coupled to a power source 1114, an ionizable media source 1116 and a precursor source. Power source 1114 includes a signal generator 1250 coupled to an amplifier 1252. The signal generator 1250 outputs a plurality of control signals to the amplifier 1252 reflective of the desired waveform. The signal generator 1250 allows for control of desired waveform parameters (e.g., frequency, duty cycle, amplitude, pulsing, etc.). In some embodiments, signal generator 1250 may pulse the waveform, e.g., a continuous-wave waveform signal may be switched on and off at a duty cycle (the duty cycle may be fixed or variable) and at a different frequency from the frequency of the continuous-wave waveform. The amplifier 1252 outputs the desired waveform at a frequency from about 0.1 MHz to about 2,450 MHz and in another embodiment from about 1 MHz to about 13.56 MHz. The power source 1114 also includes a matching network 1254 coupled to the amplifier 1252. The matching network 1254 may include one or more reactive and/or capacitive components that are configured to match the impedance of the load (e.g., plasma effluent) to the power source 1114 by switching the components or by frequency tuning.

The power source 1114 is coupled to a plasma device 1112. As shown in Fig. 15, the plasma device 1112 may be utilized for application of plasma to tissue. The device 1112 includes an inner electrode 1122, which may be an aluminum alloy rod, disposed coaxially within an outer electrode 1123. The outer electrode 1123 may be an aluminum alloy tube having an opening 1125. As shown in Fig. 14, the inner and outer electrode 1122 and 1123 are coupled to the power source 1114 via connectors 1256 and 1258, which are disposed around the inner electrode 1122 and 1123, respectively. The connectors 1256 and 1258 may be copper connector blocks.

With reference to Fig. 15, the device 1112 also includes a ceramic electrode spacer 1127 disposed between the inner and outer electrodes 1122 and 1123. The electrode spacer 1127 may be disposed at any point between the inner and outer electrodes 1122 and 1123 to provide for a coaxial configuration between the inner and outer electrodes 1122 and 1123. The electrode spacer 1127 is substantially similar to the electrode spacer 27 and may include a central opening (not explicitly shown) adapted for insertion of the inner electrode 1122 therethrough and one or more flow openings (not explicitly shown) disposed radially around the central opening to allow for the flow of plasma feedstocks through the device 1112. The electrode spacer 1127 may be frictionally fitted to the electrodes 1122 and 1123 to secure the inner electrode 1122 within the outer electrode 1123. One of the electrodes 1122 and 1123 may be an active electrode and the other may be a neutral or return electrode to facilitate in RF energy coupling.

With reference to Fig. 14, the plasma system 1100 also includes an ionizable media source 1116 and a precursor source coupled to the plasma device 1112. The ionizable media source 1116 provides ionizable feedstock, namely, helium gas, to the plasma device 1112. The ionizable media source 1116 includes a storage tank for storing the helium gas. The ionizable media source 1116 is coupled to the precursor source via tubing 1262, which includes tubing 1262a coupled to the ionizable media source 1116. The tubing 1262a branches into tubing 1262b and 1262c. The tubing 1262c is coupled to the precursor source, which may be a bubbler or a nebulizer, for aerosolizing precursor feedstocks, namely liquid hydrogen peroxide, prior to introduction thereof into the device 1112. The feedstocks are mixed upstream of the device 1112 prior to introduction thereto.

The tubing 1262b bypasses the tubing 1262c and reconnects at tubing 1262d, which is coupled to the plasma device 1112 at a coupling 1264. The coupling 1264 may be a Teflon union tee connected to the outer electrode 1123. The tubing 1262 also includes valves 1260a, 1260b, 1260c which control the flow of the helium gas and the hydrogen peroxide through the tubing 1262a, 1262b, 1262c, respectively. The tubing 1262 further includes mass flow controllers 1266b and 1266c adapted to control the flow of plasma feedstocks through the tubing 1260b and 1260c, respectively.

The system 1100 provides a flow of plasma through the device 1112 to the tissue. Plasma feedstocks, which include helium gas and hydrogen peroxide, are supplied by the ionizable media source 1116 and the precursor source, respectively, to the plasma device 1112, which are ignited to form plasma effluent containing ions, radicals, photons from the specific excited species and metastables that carry internal energy to drive desired chemical reactions with the tissue or at the surface thereof.

With reference to Fig. 16, a close-up, side view of a plasma device 1112 according to the present disclosure. Plasma device 1112 includes the inner electrode 1122 and the outer electrode 1123. The plasma device 1112 also includes a coating 1124 disposed on the outer surface of the inner electrode 1122 and on the inner surface of the outer electrode 1123. The coating 1124 is substantially similar to the coating 24 that is discussed above with respect to Figs. 4-6. In one embodiment, the plasma device 1112 may also include additional features discussed above with respect to Figs. 4-6 such as grooves disposed in a parallel or spiral configurations, nanostructure pores filled with precursor materials, and/or vents within the inner electrode 1122. In another embodiment, the inner electrode 1122 may be disposed in a variety of configurations and spatial orientation with respect to the outer electrode 1123. In particular, the inner electrode 1122 may be recessed, flush or extended relative to the outer electrode 1123 as shown in Figs. 2B - 2D. The extended distance of the inner electrode 1122 may also be adjustable as discussed above with respect to Figs. 2A - 2D.

Fig. 16 illustrates working ranges $L_{R,1}$, $L_{R,2}$ and $L_{R,3}$, and a distance $D_T$. Outer electrode 1123 includes a working range $L_{R,1}$ of energetic secondary electron emissions. Inner electrode 1122 includes working ranges $L_{R,2}$, and $L_{R,3}$ of energetic secondary electron emissions having energy $E$. In other words, the working ranges are representative of the thickness of energetic electron sheath layers 1133 and 1134, which are disposed about the inner and outer electrodes 1122 and 1123, respectively. A gap distance $\Delta$ shows the zone where the concentration of energetic secondary electrons is relatively lower. Coating the electrodes, as discussed above, reduces gap distance $\Delta$. In some embodiments, distance $\Delta$ may be reduced to zero and/or working ranges $L_{R,1}$, and $L_{R,2}$ may overlap thereby creating an hollow cathode effect. Inner electrode 1112 includes a tip 1128 having a distance $D_T$ from tissue "T". Ranges $L_{R,1}$, $L_{R,2}$ and $L_{R,3}$, indicate regions with a greatly increased concentration of electrons with relatively high energies that drive reactions in the gas phase. As discussed above, the coating 1124 on electrodes 1122 and/or 1123 can increase or enhance working ranges $L_{R,1}$ and $L_{R,2}$, and/or $L_{R,3}$ of energetic secondary electrons. Thus, varying the thickness of the coating 1124 can be used to adjust the working ranges $L_{R,1}$ and $L_{R,2}$, and/or $L_{R,3}$. Additionally or alternatively, the distance $D_T$ that tip 1128 is disposed from tissue "T" is adjustable to achieve a predetermined tissue effect (discussed in more detail below).

$$(3) \quad R(E) = \sigma(E) \cdot n_e(E) \cdot v(E).$$

Formula (3) relates the reaction rate R that indicates an inelastic (energy expending) collision where an electron at energy E, e(E), interacts with gas particle X. As a result of the collision the electron may transfer energy to X. After the collision, the electron and particle will have different energies. The rate or efficiency of this reaction is controlled by the energy dependent cross-section $\sigma(E)$ of the particular reaction.

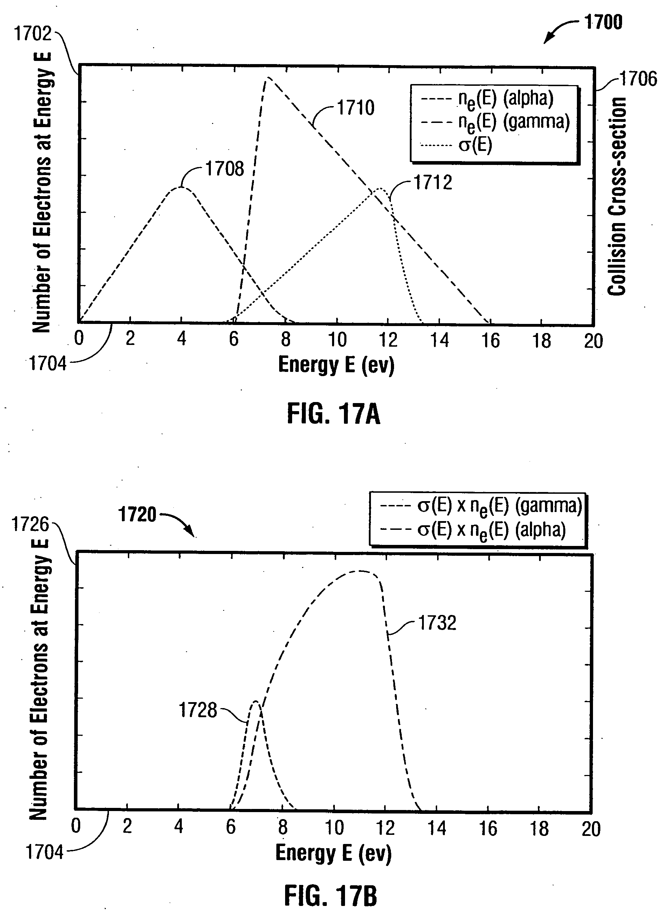

Referring now to Figs 17A and 17B that show two plots, respectively. Fig. 17A shows a plot 1700 illustrating typical electron concentrations $n_e(E)$ versus energy for alpha-mode and gamma-mode discharges, and a typical collisional reaction cross-section. Plot 1700 includes axes 1702, 1704, and 1706. Axis 1702 shows the number of electrons at energy E (i.e., a distribution). Axis 1704 shows energy E of electrons in electron-volts (eV). Line 1708 illustrates the number of electrons at energy E (axis 1702) versus energy E (axis 1704) as would be found in an alpha-mode discharge. Line 1712 illustrates the number of electrons at energy E (axis 1702) versus energy E (axis 1704) as would be found in a gamma-mode discharge, which results from enhancement of secondary emission in the gamma-mode discharge. The probability of a collisional reaction between an electron and a gas particle depends on the reaction cross-section, $\sigma(E)$, a general form of which is shown here as a function of energy E (axis 1704) as line 1710. Line 1710 shows the collision cross-section (axis 1706) versus Energy E (axis 1704).

Referring now to Fig. 17B, plot 1720 shows the calculated point-by-point multiplication product of $\sigma(E) \cdot n_e(E)$, a numeric indication of chemical reaction probability, for each mode of discharge. Plot 1702 includes an axis 1726 indicating the reaction probability at energy E. Line 1728 shows the product $\sigma(E) \cdot n_e(E)$ for an alpha-mode discharge as a function of energy E (axis 1704). Line 1732 shows the product $\sigma(E) \cdot n_e(E)$ for a gamma-mode discharge as a function of energy E (axis 1704). The overall reaction rate for each mode of discharge is related to the integral of each line (the area under each line). The reaction rate is given by the product of this quantity and the velocity distribution v(E), specifically $\sigma(E) * n_e(E) * v(E)$. The components of the product of Fig. 17B are shown in Fig. 17A. Line 1728 indicates the point-by-point multiplication (convolution) of lines 1708 and 1710, and line 1732 indicates the convolution of lines 1712 and 1710.

Referring again to Fig. 17A, line 1708 shows peak alpha electron emissions occurring at point 1708, and may correspond to an electron-voltage of about 1 eV to about 3 eV. The majority of chemical bonds and/or chemical reactions with the electrons occur within an energy range of about 2 eV to about 10 eV. Line 1710 shows the likelihood of $n_e(E)$ with additional secondary electron emissions. This illustrates that the secondary electron emissions increase the probability that chemical reactions of feedstocks with electrons to form reactive radicals occur with tissue "T" within an energy range of secondary electron emissions.

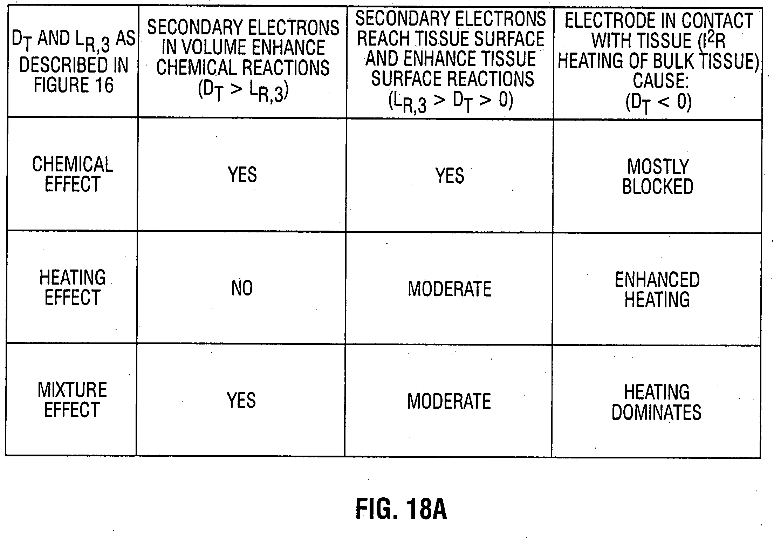

Referring again to Fig. 16, plasma device 1112 is shown with inner electrode 1128 disposed a distance $D_T$ from tissue "T". Distance $D_T$ corresponds to the magnitudes of various physicals effects, each energetic physical effect affecting directivity, selectivity, heating and other aspects of the tissue processing of tissue "T". For $D_T \gg L_{R,3}$ the secondary electrons do not reach the tissue surface. Figs. 18A - 18C include charts illustrating the contributing physical effects affecting tissue "T" as a function of distance $D_T$ that inner electrode 1128 is disposed from tissue "T" (see Fig. 16).

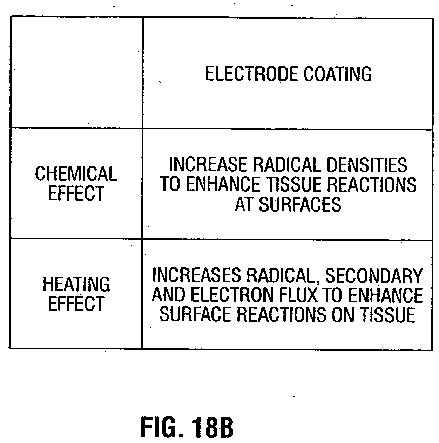

Figs. 18A and 18B illustrate the chemical effect, heating effect, and a blend of the two as a mixture effect that the plasma device 1112 has on tissue "T" (see Fig. 16). For $D_T > L_{R,3}$ more energetic secondary electrons in volume enhance chemical reactions for the chemical effect and the mixture effect, but produce minimal or no heating effect. As shown in Fig. 18A, impinging secondary electrons on the tissue surface enhance chemical reactions both in the gas volume and at the tissue surface. Secondary electron emissions also enhance tissue surface reactions when $D_T < L_{R3}$ but do not have electron stimulated surface reactions when $D_T < L_{R3}$. In summary for the energetic secondary electron emissions to enhance tissue surface reactions, the condition of formula (4) must be satisfied as follows:

$$(4) \quad 0 < D_T \leq L_{R,3}$$

Fig. 18A also shows a condition during which the inner electrode 1122 touches tissue "T" (see Fig. 16) therefore making $D_T < 0$. When the inner electrode 1122 touches tissue "T", chemical effects are mostly blocked, while bulk heating effects are enhanced. This case is mostly dominated by $I^2R$ or $j^2\rho$ (Ohmic) heating. When inner electrode 1122 touches tissue "T", the inner electrode thermally conducts heat to the tissue. Additionally, inner electrode 1122 is capacitively coupled to Tissue "T" (when touching) and electrically conducts energy thereto. Also, when inner electrode 1122 touches the tissue, the reacted tissue is moved away exposing un-reacted tissue.

With reference to Fig. 18B, a chart illustrating effects of coating is shown. Coatings enhance secondary electron emissions, thereby increasing radical fluxes and energetic electrons as well as facilitate the surface heating effect. The electrode coating 1124 increases radical densities to enhance tissue reactions at surfaces for the chemical effect. For the heating effect, the electrode coating 1124 increases radical, secondary and electron flux to enhance surface reactions on tissue.

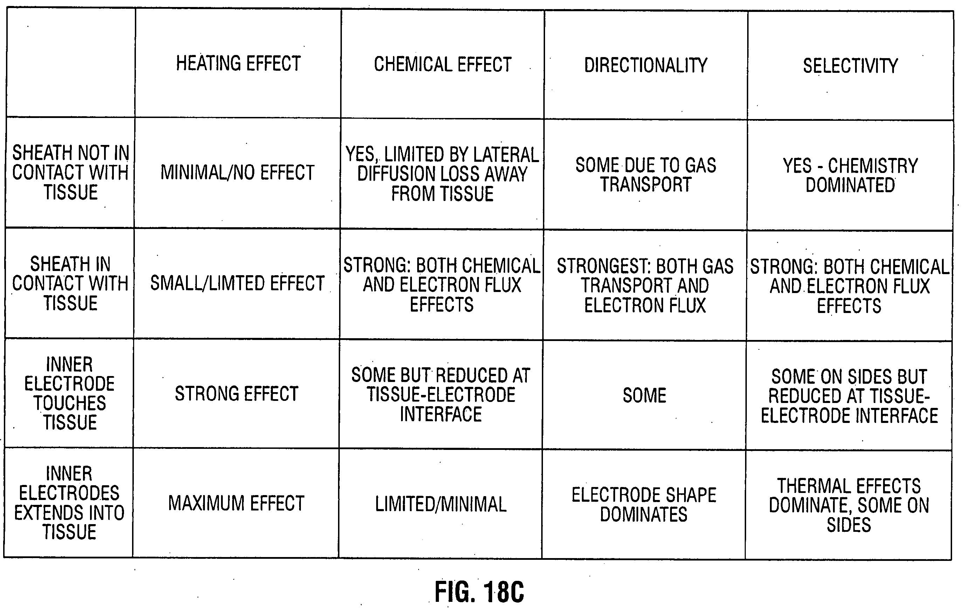

With reference to Fig. 18C, various effects of disposing inner electrode 1122 in spaced relation to tissue are illustrated. Although, Fig. 18C refers to the inner electrode 1122, in some embodiments, the outer electrode 1124 may be disposed in spaced relation to the tissue "T", both electrodes 1122 and 1124 may be disposed in spaced relation to the tissue "T", or the sheath having a working range $L_{R,1}$ may be disposed in spaced relation to tissue "T". Additionally or alternatively, one or more of plasma devices plasma device as described with reference to any one of Fig. 1 through 16 may be combined with the teachings of with Figs. 18A - 18C and may be chosen to achieve a target tissue effect or result. Anyone one or more of the chemical effect, the heating effect, the directivity, the selectivity, or any other effect as described in Fig. 18C may be selected as a desired (or target) effect, and a plasma device as described with reference to any one of Fig. 1 through 16 (or equivalents or combinations thereof), the plasma device's position in relation to tissue "T", and/or the power applied to the plasma device may be adjusted or controlled for to achieve the desired tissue effect(s).

Fig. 18C will be described as follows with reference to plasma device 1112 of Fig. 16. When the electron sheaths are not in contact with tissue "T" (e.g., the sheath having working range $L_{R,3}$) the heating effect is minimal (or no effect), the chemical effect is limited by lateral diffusion loss away from the tissue, directionality is present due to gas transport, and selectivity is present and is chemistry dominated. When the sheath is in contact with tissue, the heating effect is small or limited, the chemical effect is strong (both chemical and electron flux effects), directionality is strongest (both gas transport and electron flux), and selectivity is strong (both chemical and electron flux effects). When the inner electrode 1122 touches tissue "T," the heating effect is a strong effect, the chemical effect is present but is reduced at the tissue-electrode interface, there is some directionality, and there is some selectivity on the sides but is reduced at the tissue-electrode interface. When the center electrode (e.g., inner electrode 1122) extends into tissue, the heating effect is maximum, the chemical effect is limited (or minimal), the electrode shape dominates directionality, and for selectivity: the thermal effects dominate and there is some selectivity on the sides When the inner electrode 1122 extends into tissue or otherwise touches tissue "T", the inner electrode 1122 transfers thermal energy to the tissue and is capacitively coupled to the tissue thereby conducting electricity through tissue "T".

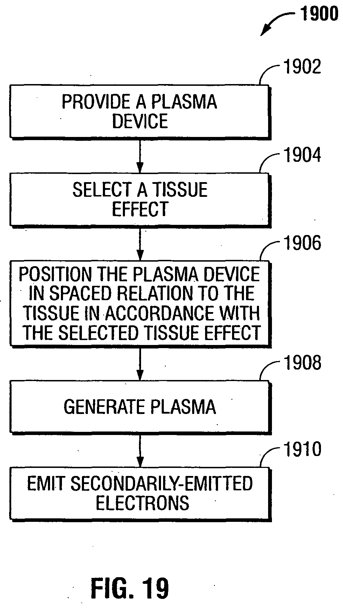

With reference to Fig. 19, a method 1900 for treating tissue is shown according to the present disclosure. Step 1902 provides a plasma device. Step 1904 selects a tissue effect. The tissue effect of step 1904 may be a heating effect, a chemical effect and/or a mixture effect as described above with respect to the Fig. 18A. Step 1906 positions the plasma device in spaced relation to the tissue in accordance with the selected tissue effect. Step 1908 generates plasma. Step 1910 emits secondarily emitted electrons via secondary electron emissions. The secondary electrons may be controlled to achieve one or more magnitudes of one or more selected tissue effects.

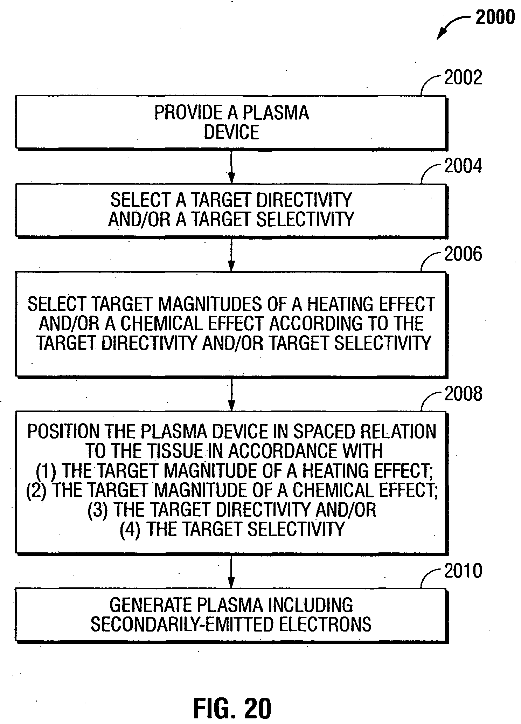

With reference to Fig. 20, a flow chart diagram of a method 2000 of plasma tissue treatment is illustrated according to the present disclosure. Step 2002 provides a plasma device. Step 2004 selects a target directivity and/or a target selectivity as described above with respect to the Fig. 18C. Directional secondary electrons predominately impinge on the bottom, as opposed to the sidewalls, of the tissue cuts. Preferential irradiation of the bottom results in a directional tissue removal. Choice of chemical radical flux and tissue type change the tissue removal rate, allowing the removal of one tissue type but not another. Selectivity between tissue types > 15 are achievable.

Step 2006 selects target magnitudes of a heating effect and/or a chemical effect according to the target directivity and/or target selectivity. Step 2008 positions the plasma device in spaced relation to tissue in accordance with (1) the target magnitude of a heating effect; (2) the target magnitude of a chemical effect; (3) the target directivity; and/or (4) the target selectivity. The selected relative magnitudes of the surface heating and chemical effects may be a function of the selected directivity and/or selectivity. Step 2010 generates a plasma including energetic secondarily emitted electrons which may ($D_T<L_{R3}$) or may not ($D_T>L_{R3}$) impinge on the tissue surface.

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope or spirit of the disclosure.

The present invention is however defined and limited only by the appended claims. Another example is overlapping working distances $L_{R1}$ and $L_{R2}$ which causes hollow cathode enhancement of the radical flux density in the volume and RF at the surface. In particular, as discussed above this allows the tailoring of the relative populations of plasma species to meet needs for the specific process desired on the workpiece surface or in the volume of the reactive plasma.

**Claims**

1. A system (10), comprising:

   a power source (14), and
   a plasma device (12;412) for use as an electrosurgical pencil, comprising:

   a dielectric spacer (432) including a substantially disk-like shape, the dielectric spacer (432) for coupling to an ionizable media source (16) configured to supply ionizable media thereto, wherein the dielectric spacer (432) is formed from a porous dielectric material configured to disperse the ionizable media;
   **characterised in that**
   the first and second electrodes (422, 423) are disposed within the dielectric spacer (432);
   wherein the first (422) and second (423) electrodes are configured to couple to the power source (14) configured to ignite the ionizable media at the plasma device (12; 412) to form a plasma effluent; and wherein one of the electrodes (422, 423) is an active electrode and the other is a neutral or return electrode to facilitate in radiofrequency (RF) energy coupling, and wherein each of the electrodes (422, 423) is coupled to the power source (14), which is configured to drive plasma generation, such that the energy from the power source (14) is used to ignite the ionizable media flowing through the plasma device (412).

2. A system according to claim 1, wherein the dielectric spacer (432) includes at least one opening (434) to provide for a flow path of the ionizable media through the dielectric spacer (432).

3. A system according to claim 1 or 2, wherein the first (422) and second (423) electrodes are substantially cylindrical rods formed from a conductive metal and disposed in parallel configuration with respect to each other and equidistant from a center of the dielectric spacer (432).

4. A system according to claim 1, 2 or 3, wherein the dielectric spacer (432) includes openings (434) through the center thereof to allow for the flow of the ionizable media therethrough.

5. The system of claim 1, wherein the dielectric spacer (432) includes openings to provide for a flow path of the ionizable media through the dielectric spacer and the openings (434) are from about 0.1 cm to about 1.0 cm at their widest thickness.

6. The system of claim 4 or 5, wherein the first and second electrodes (422, 423) have one or more regions that form and present sharpened protuberances toward the openings (434) to increase the local electric fields.

7. The system of any preceding claim, wherein the dielectric spacer (432) provides capacitive coupling between the first and second electrodes (422, 423).

8. The system of any preceding claim, comprising the ionizable media source (16) configured to supply ionizable media to the plasma device (12;412) and a precursor source (18) configured to supply precursor feedstocks to the plasma device (12;412), so as to supply a plasma feedstock to the plasma device (12;412), wherein said plasma feedstock includes the ionizable media and precursor feedstocks supplied respectively by the ionizable media source and the precursor source to the plasma device to flow through the porous dielectric media.

## Patentansprüche

1. System (10), umfassend:

   eine Stromquelle (14), und
   eine Plasmavorrichtung (12; 412) zur Verwendung als einen elektrochirurgischen Stift, umfassend:

   einen dielektrischen Abstandshalter (432), der eine im Wesentlichen scheibenartige Form einschließt, wobei der dielektrische Abstandshalter (432) zum Koppeln mit einer ionisierbaren Medienquelle (16) zum Zuführen von ionisierbaren Medien dahin konfiguriert ist, wobei der dielektrische Abstandshalter (432) aus einem porösen dielektrischen Material gebildet ist, das zum Dispergieren der ionisierbaren Medien konfiguriert ist;
   **dadurch gekennzeichnet, dass**
   die erste und die zweite Elektrode (422, 423) innerhalb des dielektrischen Abstandshalters (432) angeordnet sind; wobei die erste (422) und zweite (423) Elektrode zum Koppeln mit der Stromquelle (14) konfiguriert sind, die zum Zünden der ionisierbaren Medien an der Plasmavorrichtung (12; 412) konfiguriert ist, um einen Plasmaausfluss zu bilden; und wobei eine der Elektroden (422, 423) eine aktive Elektrode und die andere eine neutrale oder Rückelektrode ist, um die Funkfrequenz (RF)-Energiekopplung zu erleichtern, und wobei jede der Elektroden (422, 423) mit der Stromquelle (14) gekoppelt ist, die zum Antreiben einer Plasmaerzeugung konfiguriert ist, so dass die Energie von der Stromquelle (14) verwendet wird, um die ionisierbaren Medien, die durch die Plasmavorrichtung (412) strömen, zu zünden.

2. System nach Anspruch 1, wobei der dielektrische Abstandshalter (432) mindestens eine Öffnung (434) einschließt, um einen Strömungsweg der ionisierbaren Medien durch den dielektrischen Abstandshalter (432) bereitzustellen.

3. System nach Anspruch 1 oder 2, wobei die erste (422) und die zweite (423) Elektrode im Wesentlichen zylindrische Stäbe sind, die aus einem leitenden Metall gebildet sind und in paralleler Konfiguration zueinander und in gleichem Abstand von einer Mitte des dielektrischen Abstandshalters (432) angeordnet sind.

4. System nach Anspruch 1, 2 oder 3, wobei der dielektrische Abstandshalter (432) Öffnungen (434) durch seine Mitte aufweist, um die Strömung der ionisierbaren Medien dadurch zu ermöglichen.

5. System nach Anspruch 1, wobei der dielektrische Abstandshalter (432) Öffnungen einschließt, um einen Strömungsweg der ionisierbaren Medien durch den dielektrischen Abstandshalter bereitzustellen und die Öffnungen (434) von etwa 0,1 cm bis etwa 1,0 cm an ihrer größten Dicke sind.

6. System nach Anspruch 4 oder 5, wobei die erste und die zweite Elektrode (422, 423) einen oder mehrere Bereiche aufweisen, die zu den Öffnungen (434) hin scharfe Vorsprünge bilden und vorgeben, um die lokalen elektrischen Felder zu erhöhen.

7. System nach einem der vorstehenden Ansprüche, wobei der dielektrische Abstandshalter (432) eine kapazitive Kopplung zwischen der ersten und der zweiten Elektrode (422, 423) bereitstellt.

8. System nach einem der vorstehenden Ansprüche, die ionisierbare Medienquelle (16) umfassend, die zum Zuführen von ionisierbaren Medien zu der Plasmavorrichtung (12; 412) konfiguriert ist, und eine Vorläuferquelle (18), die zum Zuführen von Vorläuferausgangsmaterialien zu der Plasmavorrichtung (12; 412) konfiguriert ist, um der Plasmavorrichtung (12; 412) ein Plasmaausgangsmaterial zuzuführen, wobei das Plasmaausgangsmaterial die ionisierbaren Medien und Vorläuferausgangsmaterialien einschließt, die jeweils von der ionisierbaren Medienquelle und der Vorläuferquelle der Plasmavorrichtung zugeführt werden, um durch die porösen dielektrischen Medien zu strömen.

## Revendications

1. Système (10), comprenant :

   une source d'alimentation (14), et
   un dispositif au plasma (12 ; 412) pour son utilisation comme crayon électro-chirurgical, comprenant :

   un espaceur diélectrique (432) incluant une forme sensiblement discale, l'espaceur diélectrique (432) étant destiné à être couplé à une source de milieu ionisable (16) configurée pour fournir un milieu ionisable à celui-ci, dans lequel l'espaceur diélectrique (432) est formé d'un matériau diélectrique poreux configuré pour disperser le milieu ionisable ;

**caractérisé en ce que**

les première et deuxième électrodes (422, 423) sont disposées dans l'espaceur diélectrique (432) ;

dans lequel

les première (422) et deuxième (423) électrodes sont configurées pour coupler la source d'alimentation (14) configurée pour enflammer le milieu ionisable sur le dispositif au plasma (12 ; 412) pour former un effluent de plasma ;

et dans lequel

l'une des électrodes (422, 423) est une électrode active et l'autre est une électrode neutre ou une électrode de retour pour faciliter le couplage d'énergie de radiofréquence (RF), et dans lequel chacune des électrodes (422, 423) est couplée à la source d'alimentation (14), qui est configurée pour entraîner la génération de plasma, de telle sorte que l'énergie de la source d'alimentation (14) soit utilisée pour enflammer le milieu ionisable s'écoulant dans le dispositif au plasma (412).

2. Système selon la revendication 1, dans lequel l'espaceur diélectrique (432) inclut au moins une ouverture (434) pour fournir une voie d'écoulement du milieu ionisable dans l'espaceur diélectrique (432).

3. Système selon la revendication 1 ou 2, dans lequel les première (422) et deuxième (423) électrodes sont sensiblement des tiges cylindriques formées à partir d'un métal conducteur et disposées dans une configuration parallèle l'une par rapport à l'autre et à égale distance d'un centre de l'espaceur diélectrique (432).

4. Système selon la revendication 1, 2 ou 3, dans lequel l'espaceur diélectrique (432) inclut des ouvertures (434) dans le centre de celui-ci pour permettre l'écoulement du milieu ionisable à travers celui-ci.

5. Système selon la revendication 1, dans lequel l'espaceur diélectrique (432) inclut des ouvertures pour fournir une voie d'écoulement du milieu ionisable dans l'espaceur diélectrique et les ouvertures (434) sont d'environ 0,1 cm à environ 1,0 cm sur leur épaisseur la plus large.

6. Système selon la revendication 4 ou 5, dans lequel les première et deuxième électrodes (422, 423) ont une ou plusieurs régions qui forment et présentent des protubérances effilées en direction des ouvertures (434) pour augmenter les champs électriques locaux.

7. Système selon l'une quelconque des revendications précédentes, dans lequel l'espaceur diélectrique (432) fournit un couplage capacitif entre les première et deuxième électrodes (422, 423).

8. Système selon l'une quelconque des revendications précédentes, comprenant la source de milieu ionisable (16) configurée pour fournir un milieu ionisable au dispositif au plasma (12 ; 412) et une source de précurseur (18) configurée pour fournir des charges de précurseur au dispositif au plasma (12 ; 412), de façon à fournir une charge de plasma au dispositif au plasma (12 ; 412),

dans lequel ladite charge de plasma inclut le milieu ionisable et des charges de précurseur fournies respectivement par la source de milieu ionisable et la source de précurseur au dispositif au plasma pour s'écouler dans le milieu diélectrique poreux.

10

18 — PRECURSOR(S) SOURCE

16 — IONIZABLE MEDIA SOURCE

14 — POWER SOURCE

13b

13c

13a

PLASMA EXCITATION VESSEL

31

29

23

22

12

WORKPIECE "W"

PLASMA GENERATED SPECIES

**FIG. 1**

12

40

23

25

22

42

27

**FIG. 2A**

12

25

23

22

**FIG. 2B**

12

25

23

22

**FIG. 2C**

12

25

23

22

**FIG. 2D**

**FIG. 3**

**FIG. 4**

**FIG. 5**

**FIG. 6**

**FIG. 7**

FIG. 8

**FIG. 9**

**FIG. 10**

**FIG. 11A**

**FIG. 11B**

**FIG. 11C**

**FIG. 12A**

**FIG. 12B**

**FIG. 13**

**FIG. 14**

**FIG. 15**

EP 2 434 975 B1

**FIG. 16**

24

FIG. 17A

FIG. 17B

| $D_T$ AND $L_{R,3}$ AS DESCRIBED IN FIGURE 16 | SECONDARY ELECTRONS IN VOLUME ENHANCE CHEMICAL REACTIONS ($D_T > L_{R,3}$) | SECONDARY ELECTRONS REACH TISSUE SURFACE AND ENHANCE TISSUE SURFACE REACTIONS ($L_{R,3} > D_T > 0$) | ELECTRODE IN CONTACT WITH TISSUE ($I^2R$ HEATING OF BULK TISSUE) CAUSE: ($D_T < 0$) |
|---|---|---|---|
| CHEMICAL EFFECT | YES | YES | MOSTLY BLOCKED |
| HEATING EFFECT | NO | MODERATE | ENHANCED HEATING |
| MIXTURE EFFECT | YES | MODERATE | HEATING DOMINATES |

FIG. 18A

| | ELECTRODE COATING |
|---|---|
| CHEMICAL EFFECT | INCREASE RADICAL DENSITIES TO ENHANCE TISSUE REACTIONS AT SURFACES |
| HEATING EFFECT | INCREASES RADICAL, SECONDARY AND ELECTRON FLUX TO ENHANCE SURFACE REACTIONS ON TISSUE |

**FIG. 18B**

|  | HEATING EFFECT | CHEMICAL EFFECT | DIRECTIONALITY | SELECTIVITY |
|---|---|---|---|---|
| SHEATH NOT IN CONTACT WITH TISSUE | MINIMAL/NO EFFECT | YES, LIMITED BY LATERAL DIFFUSION LOSS AWAY FROM TISSUE | SOME DUE TO GAS TRANSPORT | YES - CHEMISTRY DOMINATED |
| SHEATH IN CONTACT WITH TISSUE | SMALL/LIMTED EFFECT | STRONG: BOTH CHEMICAL AND ELECTRON FLUX EFFECTS | STRONGEST: BOTH GAS TRANSPORT AND ELECTRON FLUX | STRONG: BOTH CHEMICAL AND ELECTRON FLUX EFFECTS |
| INNER ELECTRODE TOUCHES TISSUE | STRONG EFFECT | SOME BUT REDUCED AT TISSUE-ELECTRODE INTERFACE | SOME | SOME ON SIDES BUT REDUCED AT TISSUE-ELECTRODE INTERFACE |
| INNER ELECTRODES EXTENDS INTO TISSUE | MAXIMUM EFFECT | LIMITED/MINIMAL | ELECTRODE SHAPE DOMINATES | THERMAL EFFECTS DOMINATE, SOME ON SIDES |

**FIG. 18C**

1900

1902

```
PROVIDE A PLASMA
DEVICE
```

1904

```
SELECT A TISSUE
EFFECT
```

1906

```
POSITION THE PLASMA DEVICE
IN SPACED RELATION TO THE
TISSUE IN ACCORDANCE WITH
THE SELECTED TISSUE EFFECT
```

1908

```
GENERATE PLASMA
```

1910

```
EMIT SECONDARILY-EMITTED
ELECTRONS
```

## FIG. 19

```
                                                    2000

                                              2002
┌──────────────────────────────────────┐
│            PROVIDE A PLASMA            │
│               DEVICE                   │
└──────────────────────────────────────┘
                    │
                    ▼                         2004
┌──────────────────────────────────────┐
│        SELECT A TARGET DIRECTIVITY      │
│        AND/OR A TARGET SELECTIVITY      │
└──────────────────────────────────────┘
                    │
                    ▼                         2006
┌──────────────────────────────────────┐
│  SELECT TARGET MAGNITUDES OF A HEATING EFFECT │
│  AND/OR A CHEMICAL EFFECT ACCORDING TO THE    │
│  TARGET DIRECTIVITY AND/OR TARGET SELECTIVITY │
└──────────────────────────────────────┘
                    │
                    ▼                         2008
┌──────────────────────────────────────┐
│ POSITION THE PLASMA DEVICE IN SPACED RELATION │
│        TO THE TISSUE IN ACCORDANCE WITH       │
│  (1) THE TARGET MAGNITUDE OF A HEATING EFFECT;│
│  (2) THE TARGET MAGNITUDE OF A CHEMICAL EFFECT;│
│         (3) THE TARGET DIRECTIVITY AND/OR     │
│            (4) THE TARGET SELECTIVITY         │
└──────────────────────────────────────┘
                    │
                    ▼                         2010
┌──────────────────────────────────────┐
│        GENERATE PLASMA INCLUDING       │
│      SECONDARILY-EMITTED ELECTRONS     │
└──────────────────────────────────────┘
```

**FIG. 20**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030125727 A1